# EUROPÄISCHE PATENTSCHRIFT

(11) **EP 0 944 578 B1**
(45) Veröffentlichungstag und Bekanntmachung des Hinweises auf die Patenterteilung: **04.09.2002**
(21) Anmeldenummer: 97952849.4
(22) Anmeldetag: 01.12.1997
(51) Int. Cl.: C07C 209/36

(54) **VERFAHREN ZUR HERSTELLUNG VON AROMATISCHEN AMINEN DURCH GASPHASENHYDRIERUNG**
PROCESS FOR PRODUCING AROMATIC AMINES BY GASEOUS PHASE HYDROGENATION
PROCEDE DE PREPARATION D'AMINES AROMATIQUES PAR HYDROGENATION DANS LA PHASE GAZEUSE

(30) Priorität: 12.12.1996 DE 19651688
(43) Veröffentlichungstag der Anmeldung: 29.09.1999
(73) Patentinhaber: BAYER AG, 51368 Leverkusen (DE)
(72) Erfinder: LANGER, Reinhard, D-47800 Krefeld (DE); BUYSCH, Hans-Josef, D-47809 Krefeld (DE); GALLUS, Manfred, D-47802 Krefeld (DE); LACHMANN, Burkhard, D-40667 Meerbusch (DE); VON GEHLEN, Franz-Ulrich, D-47839 Krefeld (DE)
(86) Internationale Anmeldenummer: EP9706730
(87) Internationale Veröffentlichungsnummer: WO98025881

(56) Entgegenhaltungen:
- DE-A- 2 244 401
- DE-A- 2 849 002
- DE-A- 4 039 026

## Beschreibung

Die vorliegende Erfindung betrifft ein Verfahren zur Herstellung von aromatischen Aminen durch katalytische Hydrierung von entsprechenden aromatischen Nitroverbindungen in der Gasphase an ortsfest angebrachten Trägerkatalysatoren.

Die Hydrierung von Nitrobenzol und anderen Nitroaromaten zu den entsprechenden aromatischen Aminen in der Gasphase an ortsfest angebrachten Trägerkatalysatoren ist bekannt. So wird beispielsweise in FR 25 25 591 ein Verfahren zur Hydrierung von Nitrobenzol an stationären Kupferkatalysatoren beschrieben. Weiterhin werden in DE-A 2 135 155 und 2 244 401 Verfahren zur Reduktion von Nitroverbindungen in Gegenwart von palladiumhaltigen Trägerkatalysatoren unter Verwendung von Spinellen als Trägermaterialien beschrieben. Darüber hinaus ist aus DE-A 2 849 002 ein Verfahren zur katalytischen Hydrierung von Nitrobenzol bekannt, bei dem die Hydrierung in Gegenwart eines Mehrkomponenten-Trägerkatalysators durchgeführt wird. Nachteilig bei den in den erwähnten Patentveröffentlichungen beschriebenen Gasphasenhydrierungen ist die geringe Belastung (spezifische Belastung) der Katalysatoren. Die angegebenen bzw. berechneten Belastungen schwanken zwischen 0,2 bis 0,6 kg/l x h. Die Belastung ist dabei definiert als die Menge an Nitroaromaten in kg, die pro Liter Katalysatorschüttung innerhalb einer Stunde umgesetzt wird. Verbunden mit der geringen Katalysatorbelastung ist eine nicht befriedigende Raumzeitausbeute bei großtechnischen Verfahren zur Herstellung von aromatischen Aminen.

In der DE-A 4 039 026 wird eine Gasphasenhydrierung an hochbelastbaren Palladium-Katalysatoren beschrieben. Die Katalysatorbelastung bei diesem Verfahren liegt zwischen 0,6 und 0,95 kg/l x h. Bei der technischen Durchführung des Verfahrens hat sich jedoch gezeigt, daß der Umsatz des Nitrobenzols schon nach kurzer Zeit nur unvollständig erfolgt, so daß sich im Kondensat nicht unbedeutende Mengen an unumgesetztem Nitrobenzol neben gebildetem aromatischen Amin befinden. Um den Gehalt an Nitrobenzol unter die tolerierbare Grenze zu drücken, muß das Anilin durch aufwendige Reinigungsverfahren (Destillation) gereinigt werden. Darüber hinaus hat sich gezeigt, daß die in DE-A 4 039 026 eingesetzten Katalysatoren bei der hohen Katalysatorbelastung nur eine unbefriedigende Lebensdauer besitzen.

Aufgabe der vorliegenden Erfindung war nun ein Verfahren zur Herstellung von aromatischen Aminen durch katalytische Hydrierung der entsprechenden Nitroverbindungen in der Gasphase zur Verfügung zu stellen, das ohne Probleme in technischem Maßstab durchzuführen ist und das eine hohe Raumzeitausbeute gewährleistet, verbunden mit einer verbesserten Produktivität der eingesetzten Katalysatoren.

Dieses Ziel wurde mit dem erfindungsgemäßen Verfahren erreicht.

Gegenstand der vorliegenden Erfindung ist daher ein Verfahren zur Herstellung von aromatischen Aminen durch katalytische Hydrierung von entsprechenden aromatischen Nitroverbindungen in der Gasphase an ortsfest angebrachten Katalysatoren, die ein oder mehrere Metalle der Gruppen VIIIa, Ib, IIb, IVa, Va, VIa, IVb und Vb des Periodensystems der Elemente (Medelejew) auf einem keramischen Trägermaterial mit einer BET-Oberfläche von weniger als 40 m²/g enthalten, bei molaren Verhältnissen von Wasserstoff zu Nitrogruppe oder Nitrogruppen von 3:1 bis 30:1 und Temperaturen im Katalysatorbett von 180 bis 500°C, das dadurch gekennzeichnet ist, daß die Belastung des Katalysators an eingesetzten aromatischen Nitroverbindungen kontinuierlich oder stufenweise von 0,01 bis 0,5 auf 0,7 bis 5,0 kg/l x h erhöht wird, wobei die maximale Belastung innerhalb von 10 bis 1000 Stunden erreicht wird.

Als aromatische Nitroverbindungen können in das erfindungsgernäße Verfahren solche der nachstehenden Formel 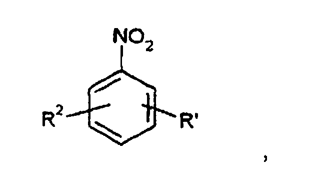 in der
- R¹ und R²: gleich oder verschieden sind und fiir C₁-C₄-Alkyl, insbesondere Methyl und Ethyl, Wasserstoff oder für die Nitrogruppe stehen,
eingesetzt werden.

Bevorzugt werden Nitrobenzol oder die isomeren Nitrotoluole, insbesondere wird Nitrobenzol in das erfindungsgemäße Verfahren zur Hydrierung eingesetzt.

Für das erfindungsgemäße Verfahren eignen sich alle Katalysatoren, die fiir die Hydrierung von Nitroverbindungen bekannt sind und bei denen die Metalle der zuvor erwähnten Haupt- und Nebengruppen des Periodensystems der Elemente auf einen keramischen Träger aufgebracht worden sind. Die Metalle können dabei in elementarer Form oder in Form einer Verbindung auf den Katalysatorträger aufgebracht worden sein. Als Metalle, die auf einen keramischen Träger aufgebracht werden können, werden insbesondere genannt Fe, Co, Pd, Pt, Cu, Ag, Zn, Ti, Zr, Hf, V, Nb, Ta, Cr, Mo, W, Ge, Sn, Pb, As, Sb, Bi, besonders bevorzugt Fe, Pd, Pt, Cu, Ti, V, Nb, Cr, Mo, Sn, Pb, Sb, Bi, ganz besonders bevorzugt Pd, V, Pb, Bi. Die Metalle können einzeln oder im Gemisch untereinander auf dem keramischen Träger aufgebracht worden sein.

Als keramische Trägermaterialien für die genannten Metalle eignen sich im Prinzip alle keramischen Feststoffe mit BET-Oberflächen von weniger als 40 m²/g, bevorzugt weniger als 20 m²/g, insbesondere weniger als 10 m²/g. Insbesondere sind als keramische Feststoffe geeignet: Metalloxide und/oder Metallmischoxide der Elemente Magnesium, Aluminium, Silicium, Germanium, Zirkonium und Titan, bevorzugt wird als Trägermaterial α-Aluminiumoxid eingesetzt.

Als ganz besonders geeignet haben sich Katalysatoren erwiesen, wie sie in DE-A 2 849 002 beschrieben sind. Hierbei sind insbesondere solche Katalysatoren zu nennen, welche vor allem Palladium, Vanadium und Blei neben anderen Metallen auf α-Aluminiumoxid schalenförmig niedergeschlagen enthalten. Besonders hervorzuheben sind daher Katalysatoren, die a) 1 bis 50 g Palladium, b) 1 bis 50 g Titan, Vanadium, Niob, Tantal, Chrom, Molybdän und/oder Wolfram und c) 1 bis 20 g Blei und/oder Wismut pro Liter oxidischen Trägermaterials enthalten, wobei das Trägermaterial eine Oberfläche von weniger als 40, bevorzugt weniger als 20, insbesondere weniger als 10 m²/g aufweist. Von den genannten Metallen des Mischkatalysators haben sich wiederum Palladium, Vanadium und Blei als besonders günstig im Einsatz erwiesen, in den zuvor angegebenen Mengen.

Die Herstellung der Katalysatoren ist aus der genannten Patentliteratur bekannt. Dabei hat es sich als Vorteil erwiesen, wenn die aktiven Komponenten des Katalysators möglichst nahe an der Trägerformkörperoberfläche als scharfe Zone niedergeschlagen vorliegen und das Innere des Trägermaterials kein Metall enthält. Die Katalysatoren können dabei mit oder ohne Vorbehandlung des Trägermaterials mit einer Base hergestellt werden.

Prinzipiell können die Trägerkatalysatoren fiir das erfindungsgemäße Verfahren jede beliebige Form aufweisen, wie Kugeln, Stäbchen, Raschigringe, Granulat oder Tabletten. Bevorzugt werden Formkörper benutzt, deren Schüttungen einen niedrigen Strömungswiderstand bei gutem Gas-Oberflächen-Kontakt aufweisen, wie Raschigringe, Sattelkörper, Wagenräder und/oder Spiralen. Bei dem erfindungsgemäßen Verfahren ist es möglich, daß die Katalysatorschüttung in den Reaktoren nur aus dem Trägerkatalysator besteht oder zusätzlich mit inertern Trägermaterial oder anderen inerten Füllkörpern, wie z.B. Glas oder Keramik verdünnt wird. Die Katalysatorschüttung kann bis zu 90 Gew.-%, bevorzugt bis zu 75 Gew.-%, insbesondere bis zu 50 Gew.-% aus inerten Füll- oder Trägermaterial bestehen. Die Schüttung kann dabei einen Verdünnungsgradienten aufweisen der Art, daß die Verdünnung in Strömungsrichtung abnimmt. Die Schüttung kann an der Aufströmoberfläche zwischen 10 bis 50 Gew.-% Füllmaterial enthalten und am Ausströmende 80 bis 100 Gew.-% aus reinem Trägerkatalysator bestehen.

Als Reaktoren können fiir das erfindungsgemäße Verfahren alle bekannten Reaktoren eingesetzt werden, die für Gasphasenreaktionen mit gekühlten stationären Katalysatorschüttungen geeignet sind. Geeignet sind z.B. Rohrbündelreaktoren, in denen sich der Katalysator innerhalb von Wärmeträger umspülten Rohren befindet und Reaktoren, in denen umgekehrt der Wärmeträger innerhalb der Rohre fließt und der Katalysator sich außerhalb der Rohre befindet. Beispielsweise sind solche Reaktoren aus DE-A 2 848 014 und 3 007 202 bekannt.

Als besonders vorteilhaft haben sich Reaktoren fiir das erfindungsgemäße Verfahren erwiesen, in denen der Wärmeträger innerhalb der Rohre fließt und der Katalysator sich außerhalb der Wärmeträgerrohre befindet (Reaktoren vom Lindetyp). In diesen Reaktoren werden, relativ zu konventionellen Rohrbündelreaktoren, über viele Regenerationszyklen konstante Laufzeiten zwischen den Regenerierungen beobachtet.

Die Länge der Katalysatorschüttung in Strömungsrichtung beim erfindungsgemäßen Verfahren liegt bei 0,5 bis 20, bevorzugt 1 bis 10, besonders bevorzugt bei 2 bis 6 m.

Das erfindungsgenäße Verfahren wird bevorzugt durchgeführt bei molaren Verhältnissen von Wasserstoff zu Nitrogruppe oder Nitrogruppen der eingesetzten Nitroverbindung von 4:1 bis 20:1, insbesondere 5:1 bis 10:1.

Die Wasserstoffkonzentration kann durch Zumischen von inerten Trägergasen wie z.B. Stickstoff, Helium, Argon und/oder Wasserdampf herabgesetzt werden. Bevorzugt wird Stickstoff eingesetzt. Pro Mol Wasserstoff können bis zu 10 Mol, bevorzugt bis zu 3 Mol insbesondere bis zu 1 Mol inertes Trägergas zugespeist werden.

Die Verdünnung mit inertem Trägergas wird bevorzugt zu Beginn einer Fahrperiode mit frischem Katalysator und nach dem Regenerieren des Katalysators durch Abbrennen mit Luft und Reduzieren mit Wasserstoff angewendet. Bevorzugt wird in den ersten 300 Stunden, besonders bevorzugt in den ersten 200 Stunden, insbesondere in den ersten 100 Stunden nach dem Wiederanfahren mit Inertgas verdünnt.

Die Regenerierung desaktivierter Schüttungen erfolgt mit N₂/O₂-Mischungen bei Temperaturen zwischen 200 und 400°C, bevorzugt zwischen 250 und 350°C am Kontakt, dabei wird bevorzugt mit N₂-Gehalten zwischen 90 und 99 % im Gastrom begonnen und der O₂-Gehalt während des Abbrennens schrittweise auf den reiner Luft angehoben, ggf. können am Ende der Regenerierung mit reinem Sauerstoff hartnäckige Verkokungen abgebrannt werden. Anstelle von Stickstoff können auch andere inerte Trägergase zu Sauerstoff oder Luft zugemischt werden, wie z.B. Argon, Helium oder Wasserdampf.

Das erfindungsgemäße Verfahren wird bevorzugt bei Temperaturen im Bereich von 180 bis 500, besonders bevorzugt 200 bis 460, insbesondere 220 bis 440°C durchgeführt. Dabei kann es von Vorteil sein, wenn die Temperatur des Kühlmediums im erfindungsgemäßen Verfahrens während eines Betriebszyklus kontinuierlich oder schrittweise angehoben wird.

Das erfindungsgemäße Verfahren wird bei einem Druck von 0,5 bis 5, bevorzugt 1 bis 3 bar betrieben.

Für das erfindungsgemäße Verfahren ist es wesentlich, daß die Belastung des Katalysators an eingesetzten aromatischen Nitroverbindungen innerhalb eines gewissen Zeitraums kontinuierlich oder stufenweise auf den gewünschten Belastungswert erhöht wird. Bevorzugt ist daher eine Arbeitsweise in der die Belastung des Katalysators innerhalb von 20 bis 500, insbesondere 30 bis 300, ganz besonders bevorzugt 40 bis 200 Stunden kontinuierlich oder stufenweise von 0,01 bis 0,5 , bevorzugt 0, 1 bis 0,4, insbesondere 0,15 bis 0,3 auf 0,7 bis 5,0, insbesondere von 0,8 bis 3,0, ganz besonders bevorzugt 1,0 bis 2,0 kg/l x h erhöht wird.

Die hohe Endbelastung wird bis zum Durchbruch von unumgesetztem Edukt konstant gehalten. Wenn die Eduktkonzentration am Reaktorende einen zu hohen Wert annimmt kann die Temperatur des Wärmeträgers angehoben werden und/oder die Belastung an Edukt gesenkt werden um eine Produktionsunterbrechung zur Regeneration des Katalysators herauszuzögern.

In einer speziellen Ausgestaltungsform des erfindungsgemäßen Verfahrens werden Schüttungen eingesetzt, in denen die zuvor beschriebenen Trägerkatalysatoren im Gemisch mit einem weiteren festen Katalysator eingesetzt werden, bei dem Palladium auf Graphit oder graphithaltigem Koks als Trägermaterial aufgebracht wurde und bei dem das Trägermaterial eine BET-Oberfläche von 0,05 bis 10 m²/g, bevorzugt 0,2 bis 5 m²/g besitzt. Der Palladiumgehalt des graphitgeträgerten Katalysators liegt bei 0,1 bis 7 Gew.-%, bevorzugt bei 1,0 bis 6 Gew.-%, besonders bevorzugt bei 1,5 bis 5 Gew.-%, ganz besonders bevorzugt bei 2 bis 4 Gew.-%.

Werden die Trägerkatalysatoren auf Basis von keramischem Trägermaterial und die Trägerkatalysatoren auf Basis von Graphit als Trägermaterial in Mischungen untereinander eingesetzt, so beträgt das Verhältnis der Gewichte in der Mischung 1/1 bis 1000/1, bevorzugt 10/1 bis 100/1, insbesondere 90/1 bis 99/1 (keramischer Trägerkatalysator zu graphitischen Trägerkatalysator).

Der graphitische Katalysator wird bevorzugt im ersten Drittel der Katalysatorschüttung eingesetzt, kann aber auch gleichmäßig über die gesamte Schüttung verteilt sein.

Die technische Verwirklichung des erfindungsgemäßen Verfahrens kann beispielsweise wie folgt erfolgen: Ein Kreisgastrom im wesentlichen bestehend aus Wasserstoff und wenig Wasser wird verdichtet, um die Strömungswiderstände der Anlage zu überwinden. Mittels Gegenstromwärmetauschung wird der Gasstrom aufgeheizt, wobei die Wärme z.B. den Kreisgasstrom vor der Kondensation der Produkte entnommen wird. Der Kreisgastrom wird auf die gewünschte Temperatur gebracht. Im Frischwasserstoff der den verbrauchten ersetzt, wird der zu hydrierende Nitroaromat verdampft, überhitzt und anschließend werden die beiden Gasströme vermischt. Das Gasgemisch wird in einem thermostatisierten Reaktor mit stationär angeordneten Katalysator eingeleitet. Die freiwerdende Reaktionswärme wird mittels eines Wärmeträgers dem Reaktor entnommen. Der den Reaktor verlassende Produktstrom wird zum Aufheizen des Kreisgasstromes genutzt und dann bis zur Kondensation von gebildetetem Anilin mit Wasser abgekühlt. Die Flüssigkeiten werden ausgeschleust, ebenso eine kleine Menge Kreisgas zum Entfernen von Gasen, z.B. Stickstoff, die sich sonst anreichern würden. Das Kreisgas wird anschließend wieder dem Verdichter zugeführt.

In einer bevorzugten Ausgestaltung des erfindungsgemäßen Verfahrens wird die Katalysatorschüttung in einen Reaktor vom Lindetyp (Wärmeträger fließt innerhalb der Reaktorrohre und der Katalysator ist außerhalb der Wärmeträgerrohre angeordnet) eingebracht und wie oben beschrieben verfahren. Dabei wird frischer oder frisch regenerierter Katalysator in den ersten Stunden mit Stickstoff-Wasserstoff-Mischungen betrieben. Die Vorteile dieser bevorzugten Betriebsweise sind: hohe Selektivitäten und lange Zeiträume zwischen den Regenerierungen auch nach vielen Produktionszyklen.

Das erfindungsgemäße Verfahren zeichnet sich insbesondere durch die erzielbaren hohen Raumzeitausbeuten aus, verbunden mit einer Verringerung der benötigten Apparategrößen und einer deutlich erhöhten Produktivität der Katalysatoren. Damit gelingt durch das erfindungsgemäße Verfahren eine erhebliche Produktionssteigerung in bestehenden Anlagen.

Insbesondere erweist sich die Ausgestaltung des erfindungsgemäßen Verfahrens vorteilhaft bei Verwendung von Trägerkatalysatoren mit keramischen Trägermaterialien in Kombination mit Trägerkatalysatoren mit Graphit als Träger unter Verwendung eines Reaktortyps, bei dem die Trägerkatalysatoren sich außerhalb der das Wärmeträgeröl enthaltenden Rohre befindet (Linde-Typ).

### Beispiele

### Katalysatorpräparation auf keramischem Träger.

### Beispiel 1

Ein Liter eines α-Al2O3 -Trägers in Kugelform mit 3 bis 5 Millimetern Durchmesser, einer BET-Oberfläche von 9,8 m2/g, einer Saugfähigkeit von 45,1 ml Wasser pro 100 g Träger und einem Schüttgewicht von 812 g/L wurde mit 366 ml einer 10,8 g (entsprechend 0,27 Grammäquivalenten) NaOH enthaltenden wässrigen Lösung imprägniert. Die Lösung wurde innerhalb weniger Minuten völlig vom Träger aufgenommen.

Der feuchte Träger wurde in einem warmen aufsteigenden starken Luftstrom getrocknet. Die Trockenzeit bis zur Gewichtskonstanz betrug ungefähr 15 Minuten. Der Restfeuchtegehalt lag nach dem Abkühlen etwa bei 1% der Saugfähigkeit des Trägers.

Der so vorbehandelte trockene Träger wurde seiner Saugfähigkeit entsprechend mit 366 ml einer wässrigen Natriumtetrachloropalladat-Lösung, die 9 g Palladium (entsprechend 0,169 Grammäquivalenten) enthielt, getränkt und 15 Minuten stehen gelassen. Zur Reduktion der auf dem Träger abgeschiedenen Palladiumverbindung zu metallischem Palladium wurde der Katalysator mit 400 ml einer 10 %igen wässigen Hydrazinhydrat-Lösung überschichtet und 2 Stunden stehen gelassen. Danach wurde der Katalysator mit vollentsalztem Wasser gründlich gespült, bis keine Ionen der bei der Katalysatorherstellung verwendeten Verbindungen im Waschwasser mehr nachweisbar waren, was nach ca. 10 Stunden der Fall war.

Anschließend wurde wieder in einem starken warmen aufsteigenden Luftstrom bis zur Gewichskonstanz getrocknet.

Der Pd-haltige Katalysator wurde anschließend mit 366 ml einer wäßrigen, 9 g Vanadium als Vanadyloxalat enthaltenden Lösung getränkt. Die Trockung des Trägers im Warmluftstrom erfolgte wie oben angegeben. Anschließend wurde der Katalysator in einem Rohrofen bei 300°C 6 Stunden thermisch behandelt, wobei das Oxalat zersetzt wurde.

Abschließend wurde der Katalysator mit 366 ml einer wäßrigen, 3 g Blei als Bleiacetat enthaltenden Lösung getränkt und wiederum im aufsteigenden Luftstrom getrocknet.

Der fertige Katalysator enthielt 9 g Pd, 9 g Vanadium und 3 g Blei und entsprach dem Katalysator aus Beispiel 1 in DE-OS 2849002.

### Beispiele mit Katalysatorschüttungen die keramische Trägerkatalysatoren enthalten.

### Beispiel 2 (Vergleichsbeispiel 1)

In ein mit Öl thermostatisiertes Reaktorrohr mit einem Innendurchmesser von ca. 26 mm wurde eine 285 cm hohe Schüttung eines Katalysator hergestellt gemäß Beispiel 1 gefüllt. Der Katalysator wurde zuerst mit Stickstoff, dann mit Wasserstoff gespült und anschließend in einem Wasserstoffstrom von ca. 770 NL/h in 5 Stunden auf 240°C aufgeheizt. Nun wurde begonnen Nitrobenzol im Wasseistoffstrom zu verdampfen. Die Nitrobenzol-Wasserstoff-Mischung gelangte mit ca. 230°C auf die Stirnfläche der Katalysatorschüttung. Die spezifische Belastung des Katalysators wurde innerhalb von 48 Stunden von 0,1 auf 0,47 kg/L×h erhöht, was einer Flächenbelastung von 1425 kg/m2×h entspricht, so daß im Mittel eine Belastung von 0,46 kg/L×h erzielt wurde. Während des gesamten Versuches wurde die Temperatur in der gesamten Schüttung verfolgt und darauf geachtet, daß die Katalysatortemperatur an keiner Stelle oberhalb 440°C lag.

Die Öltemperatur wurde in 20°C-Schritten nach ca. 700, 800 und 900 Stunden von 240°C auf 300°C erhöht. Die Änderung der Öltemperatur entlang des Reaktorrohres betrug ca. ±1°C. Die Strömungsgeschwindigkeit des Öles entlang der Rohroberfläche betrug ungefähr 1,5 m/s,

Der Katalysator erreichte eine Standzeit von ca. 1450 Stunden, nach denen der Nitrobenzol-Gehalt des Kondensates von 0 auf ca. 300 ppm anstieg. Daraufhin mußte der Katalysator durch Abbrennen regeneriert werden. Damit hat der Katalysator unter den angegebenen Reaktionsbedingungen eine Produktivität von ca. 670 kg/L erreicht.
Die durchschnittliche Selektivität betrug 98,4%.

Im zweiten Zyklus nach der Regenerierung verhielt sich der Katalysator mit einer Standzeit von 1400 Stunden und 98,6 % Selektivität unverändert.

### Beispiel 3

In ein mit Öl thermostatisiertes Reaktorrohr mit einem Innendurchmesser von ca. 26 mm wurde eine 285 cm hohe Schüttung eines Katalysator hergestellt gemäß Beispiel 1 gefüllt. Der Katalysator wurde zuerst mit Stickstoff, dann mit Wasserstoff gespült und anschließend in einem Wasserstoffstrom von ca. 1528 NL/h in 5 Stunden auf 240°C aufgeheizt. Anschließend wurde begonnen, Nitrobenzol im Wasserstoffstrom zu verdampfen. Die Nitrobenzol-Wasserstoff-Mischung gelangte mit ca. 230°C auf die Oberfläche der Katalysatorschüttung. Die spezifische Belastung des Katalysators wurde innerhalb von 80 Stunden von 0,2 auf 1,05 kg/L×h erhöht, was einer Flächenbelastung von 2994 kg/m2×h entspricht, so daß im Mittel eine Belastung von 1,03 kg/L×h erzielt wurde. Im gesamten Versuch wurde darauf geachtet, daß der Katalysator an keiner Stelle wärmer als 440°C wird.

Die Öltemperatur wurde in 20°C-Schritten nach ca. 700, 800 und 900 Stunden von 240°C auf 300°C erhöht. Die Änderung der Öltemperatur entlang des Reaktorrohres betrug ca. ±1°C. Die Strömungsgeschwindigkeit des Öles entlang der Rohroberfläche betrug ungefähr 1,5 m/s.

Der Katalysator erreichte eine Standzeit von ca. 1050 Stunden, nach denen der Nitrobenzol-Gehalt des Kondensates von 0 auf ca. 300 ppm anstieg und der Katalysator daraufhin durch Abbrennen regeneriert werden mußte. Damit hat der Katalysator unter den angegebenen Reaktionsbedingungen eine Produktivität von ca. 1080 kg/L erreicht und ist ca. 1,6 mal produktiver als im Vergleichsbeispiel.

Die durchschnittliche Selektivität betrug 99,0% und liegt um 0,6% über der des Vergleichversuches.

Im zweiten Zyklus nach der Regenerierung verhielt sich der Katalysator mit einer Standzeit von 990 Stunden und 99,2 % Selektivität unverändert.

Da die Zeit für die Regeneration des Katalysators mit einigen Stunden relativ zur Laufzeit eines Zyklus kaum ins Gewicht fällt ist durch die hohe Belastung die produzierte Anilinmenge pro Zeiteinheit doppelt so groß wie die im Vergleichsbeispiel.

### Katalysatorpräparation auf Graphitträger.

Als Trägermaterial wurde Graphitgranulat EG 17 der Firma Ringsdorff benutzt, das eine BET-Oberfläche von ca. 0,4-0,8 m2/g besitzt.
Die Korngrößen lagen zwischen 4 und 5 mm.

Die Beispiele sollen in keiner Weise einschränkend verstanden werden, auch mit anderen Graphiten und graphithaltigen Materialien mit geringer BET-Oberfläche werden ähnliche Ergebnisse erzielt.

### Die Katalysatorpräparationen erfolgten in folgender Weise:

Graphitgranulat EG 17 der Firma Ringsdorf (Granulat 4-5 mm, Rüttelgewicht 650-1000 g/ml) mit einer Saugfähigkeit von 7 ml Acetonitril pro 100 g Träger werden in einem drehbaren Gefäß vorgelegt und unter Drehen mit einer Lösung aus Palladiumacetat in Acetonitril versetzt. Die Mischung wird bis zur vollständigen Aufnahme der Lösung durch den Träger bewegt. Darauf folgt eine 5 minütige Trocknung des Feststoffes in einem starken aufsteigenden 40°C warmen Luftstrom. Die Schritte Tränken und Trocknen werden so oft wiederholt, bis die gewünschte Menge an Palladium aufgebracht ist.

Der getrocknete Katalysator wird anschließend in einem heißen Wasserstoffstrom bei Normaldruck aktiviert.

### Beispiel 4

0,6% Pd auf EG17
2000 g Träger
3 Tränkungen mit je
8,3 g PdAc2 in 140 g Acetonitril
20 h bei 370°C im H2-Strom aktiviert

### Beispiel 5

2,4% Pd auf EG17
2000 g Träger
10 Tränkungen mit je
10 g PdAc2 in 140 g Acetonitril
20 h bei 370°C H2-Strom aktiviert

### Beispiel mit Katalysatorschüttung die graphitischen Trägerkatalysator und inerten Träger enthält.

### Beispiel 6 (Vergleichsbeispiel 2)

In ein mit Öl thermostatisiertes Reaktorrohr mit einem Innendurchmesser von ca. 26 mm wurde eine 285 cm hohe Schüttung eines Katalysator hergestellt gemäß Beispiel 4 gefüllt. Die Katalysatorschüttung war in 6 ca. 47,5 cm lange Abschnitte unterteilt in denen der Katalysator mit unbehandeltem Trägermaterial homogen verdünnt eingefüllt wurde. Die Verdünnung nahm in Strömungsrichtung wie folgt ab: 97%, 94%, 88%, 60%, 60%, 0%. Im letzten Abschnitt befand sich demnach unverdünnter Katalysator.

Die Schüttung wurde zuerst mit Stickstoff, dann mit Wasserstoff gespült und anschließend in einem Wasserstoffstrom von ca. 1528 NL/h in 5 Stunden auf 240°C aufgeheizt. Anschließend wurde begonnen Nitrobenzol im Wasserstoffstrom zu verdampfen. Die Nitrobenzol-Wasserstoff-Mischung gelangte mit ca. 230°C auf die Oberfläche der Katalysatorschüttung. Die spezifische Belastung des Katalysators wurde innerhalb von 60 Stunden von 0,4 auf 0,84 kg/L×h erhöht, was einer Flächenbelastung von 2395 kg/m2×h entspricht. Im gesamten Versuch wurde darauf geachtet, daß der Katalysator an keiner Stelle wärmer als 440°C wird.

Die Änderung der Öltemperatur entlang des Reaktorrohres betrug ca. ±1°C. Die Strömungsgeschwindigkeit des Öles entlang der Rohroberfläche betrug ungefähr 1,5 m/s.

Der Katalysator erreichte eine Standzeit von ca. 70 Stunden, nach denen der Nitrobenzol-Gehalt des Kondensates von 0 auf ca. 300 ppm anstieg. Damit hat der Katalysator unter den angegebenen Reaktionsbedingungen eine Produktivität von ca. 50 kg/L erreicht.
Die durchschnittliche Selektivität betrug 99,5%.

Durch Anheben der Temperatur des Wärmeträgers von 240 auf 260 und 300°C konnte dem Nitrobenzoldurchbruch nicht begegnet werden, der rasch auf mehr als 10% anstieg.

### Beispiele mit Katalysatorschüttungen die sowohl keramische als auch graphitische Trägerkatalysatoren enthalten.

### Beispiel 7

In ein mit Öl thermostatisiertes Reaktorrohr mit einem Innendurchmesser von ca. 26 mm wurde eine 285 cm hohe Schüttung aus Katalysatoren hergestellt gemäß Beispiel 4 und 1 gefüllt. Die Schüttung bestand aus zwei Abschnitten von ca. 50 und 235 cm Länge.

Der vordere 50 cm lange Bereich enthielt Katalysator aus Beispiel 1 zu 98% mit unbehandeltem Trägermaterial verdünnt. Der hintere 235 cm lange Bereich enthielt unverdünnten Katalysator aus Beispiel 1.

Die Schüttung wurde zuerst mit Stickstoff, dann mit Wasserstoff gespült und anschließend in einem Wasserstoffstrom von ca. 1528 NL/h in 5 Stunden auf 240°C aufgeheizt. Anschließend wurde begonnen Nitrobenzol im Wasserstoffstrom zu verdampfen. Die Nitrobenzol-Wasserstoff-Mischung gelangte mit ca. 230°C auf die Oberfläche der Katalysatorschüttung. Die spezifische Belastung des Katalysators wurde innerhalb von 70 Stunden von 0,6 auf 0,9 und in weiteren 140 Stunden auf 1,06 kg/L×h erhöht, was einer Flächenbelastung von 3081 kg/m2×h entspricht, so das im Mittel eine Belastung von 1,04 kg/L×h erzielt wurde. Im gesamten Versuch wurde darauf geachtet, daß der Katalysator an keiner Stelle wärmer als 440°C wird.

Die Öltemperatur wurde nach 140 Stunden von 240°C auf 300°C erhöht. Die Änderung der Öltemperatur entlang des Reaktorrohres betrug ca. ±1°C. Die Strömungsgeschwindigkeit des Öles entlang der Rohroberfläche betrug ungefähr 1,5 m/s.

Die Katalysatorschüttung erreichte eine Standzeit von ca. 1450 Stunden, nach denen der Nitrobenzol-Gehalt des Kondensates von 0 auf ca. 300 ppm anstieg und der Katalysator daraufhin durch Abbrennen regeneriert werden mußte. Damit hat die Schüttung unter den angegebenen Reaktionsbedingungen eine Produktivität von ca. 1508 kg/L erreicht und ist ca. 2,3 mal produktiver als die Schüttung im Vergleichsbeispiel 1 und ca. 30 mal produktiver als die Schüttung im Vergleichsbeispiel 2.
Die durchschnittliche Selektivität betrug 99,3%.

### Beispiel 8

In ein mit Öl thermostatisiertes Reaktorrohr mit einem Innendurchmesser von ca. 26 mm wurde eine 285 cm hohe Schüttung aus Katalysatoren hergestellt gemäß Beispiel 5 und 1 gefüllt. Die Schüttung bestand aus einer innigen Mischung der Katalysatoren, die zu 3% Katalysator aus Beispiel 5 und zu 97% Katalysator aus Beispiel 1 enthielt.

Die Schüttung wurde zuerst mit Stickstoff, dann mit Wasserstoff gespült und anschließend in einem Wasserstoffstrom von ca. 1528 NL/h in 5 Stunden auf 240°C aufgeheizt. Anschließend wurde begonnen Nitrobenzol im Wasserstoffstrom zu verdampfen. Die Nitrobenzol-Wasserstoff-Mischung gelangte mit ca. 230°C auf die Oberfläche der Katalysatorschüttung. Die spezifische Belastung des Katalysators wurde innerhalb von 280 Stunden von 0,25 auf 1,07 erhöht, was einer Flächenbelastung von 3110 kg/m2×h entspricht, so das im Mittel eine Belastung von 0,98 kg/L×h erzielt wurde. Im gesamten Versuch wurde darauf geachtet, daß der Katalysator an keiner Stelle wärmer als 440°C wird.

Die Öltemperatur wurde in 20°C Schritten nach 460, 1000 und 1100 Stunden von 240°C auf 300°C erhöht. Die Änderung der Öltemperatur entlang des Reaktorrohres betrug ca. ±1°C. Die Strömungsgeschwindigkeit des Öles entlang der Rohroberfläche betrug ungefähr 1,5 m/s.

Der Katalysator erreichte eine Standzeit von ca. 1400 Stunden, nach denen der Nitrobenzol-Gehalt des Kondensates von 0 *auf* ca. 300 ppm anstieg und der Katalysator daraufhin durch Abbrennen regeneriert werden mußte. Damit hat die Schüttung unter den angegebenen Reaktionsbedingungen eine Produktivität von ca. 1372 kg/L erreicht und ist ca. 2 mal produktiver als die Schüttung im Vergleichsbeispiel 1 und ca. 27 mal produktiver als die Schüttung im Vergleichsbeispiel 2.
Die durchschnittliche Selektivität betrug 99,0%.

### Vergleich zwischen Rohrbündelreaktor und Lindereaktor.

### Beispiel 9 (Rohrbündelreaktor)

Der Versuch aus Beispiel 3 wurde in einem 55 Rohre enthaltenden Rohrbündelreaktor wiederholt. Die Rohre hatten einen Innendurchmesser von ca. 26 mm, eine Wandstärke von ca. 2 mm und eine Länge von ca. 3,5 m.

In jedes Reaktorrohr wurde eine ca. 285 cm lange Katalysatorschüttung gefüllt, ingesamt ca. 83 Liter. Die restliche Schüttung bestand aus inerten Füllkörpern.
Die gesamte Aufströmfläche der Schüttung betrug ca. 292 cm².
In diesem Reaktor wurde Beispiel 3 über 10 Produktionszyklen wiederholt.
Regeneriert wurde über 10 bis 15 Stunden mit N₂-Luft-Mischungen und mit reiner Luft bei einer Wärmeträgertemperatur von 290 bis 320 °C.
Die durchschnittlichen Selektivitäten lagen zwischen 99,0 und 99,5%.
Die Standzeiten der einzelnen Produktionszyklen sind in der Tabelle 1 angegeben.

### Beispiel 10 (Lindereaktor)

Der Versuch aus Beispiel 3 wurde in einem 55 Rohre enthaltenden Rohrbündelreaktor wiederholt. Die Rohre hatten einen Innendurchmesser von ca. 26 mm, eine Wandstärke von ca. 2 mm und eine Länge von ca. 3,5 m. Der Reaktor hatte einen Innendurchmesser von ca. 295 mm.

Die Katalysatorschüttung von ca. 83 Litern wurde mit einer Höhe von ca. 285 cm in den Zwischenräumen zwischen den Rohen angebracht und besaß eine Aufströmoberfläche von ca. 292 cm².

Oberhalb und unterhalb der Katalysatorschüttung befanden sich Schüttungen aus Maschendrahtringen. Das Kühlmedium wurde durch die Rohre geführt.

In diesem Reaktor wurde Beispiel 3 über 10 Produktionszyklen wiederholt.
Die durchschnittlichen Selektivitäten lagen zwischen 99,1 und 99,4%.

Die Standzeiten der einzelnen Produktionszyklen sind in der Tabelle 1 angegeben.

**Tabelle 1:**

| **Standzeiten der Produktionszyklen der Beispiele 9 und 10 in Stunden.** | | |
|---|---|---|
| Produktionszyklus | Standzeit (Beispiel 9) | Standzeit (Beispiel 10) |
| 1 | 1010 | 950 |
| 2 | 970 | 930 |
| 3 | 940 | 910 |
| 4 | 910 | 900 |
| 5 | 870 | 890 |
| 6 | 840 | 880 |
| 7 | 810 | 870 |
| 8 | 750 | 860 |
| 9 | 710 | 850 |
| 10 | 650 | 840 |

## Patentansprüche

1. Verfahren zur Herstellung von aromatischen Aminen durch katalytische Hydrierung von entsprechenden aromatischen Nitroverbindungen in der Gasphase an ortsfest angebrachten Katalysatoren, die ein oder mehrere Metalle der Gruppen VIIIa, Ib, IIb, IVa, Va, VIa, IVb und Vb des Periodensystems der Elemente (Medelejew) auf einem keramischen Trägermaterial mit einer BET-Oberfläche von weniger als 40 m²/g enthalten, bei molaren Verhältnissen von Wasserstoff zu Nitrogruppe oder Nitrogruppen von 3:1 bis 30:1 und Temperaturen im Katalysatorbett von 180 bis 500°C, **dadurch gekennzeichnet, daß** die Belastung des Katalysators an eingesetzten aromatischen Nitroverbindungen kontinuierlich oder stufenweise von 0,01 bis 0,5 auf 0,7 bis 5,0 kg/l x h erhöht wird, wobei die maximale Belastung innerhalb von 10 bis 1000 Stunden erreicht wird.

2. Verfahren zur Herstellung von aromatischen Aminen nach Anspruch 1, **dadurch gekennzeichnet, daß** das Verfahren in Reaktoren durchgeführt wird, bei denen sich der Wärmeträger innerhalb der Rohre befindet und der Katalysator außerhalb der den Wärmeträger enthaltenden Rohre angeordnet ist.

3. Verfahren zur Herstellung von aromatischen Aminen nach Anspruch 1, **dadurch gekennzeichnet, daß** als Katalysator ein Trägerkatalysator verwendet wird, bei dem Palladium auf Graphit oder graphithaltigen Koks als Träger aufgebracht wurde, der eine BET-Oberfläche von 0,05 bis 10 m²/g besitzt und dessen Gehalt an Palladium 0,1 bis 7 Gew.-% beträgt, bezogen auf das Katalysatorgewicht, in Kombination mit einem Katalysator, der eines oder mehrere Metalle der Gruppen VIIIa, Ib, IIb, IVa, Va, VIa, IVb und Vb auf einem keramischen Trägermaterial mit einer BET-Oberfläche kleiner 40 m²/g enthält, und wobei das Mengenverhältnis von keramischem Trägerkatalysator zu graphitischem Trägerkatalysator zwischen 1/1 und 1/1000 Gewichtsteilen beträgt.

4. Verfahren zur Herstellung von aromatischen Aminen nach Anspruch 1-3, **dadurch gekennzeichnet, daß** die Katalysatorschüttungen durch inerte Füllkörper verdünnt sind und ggf. Aktivitätsgradienten aufweisen.

## Claims

1. Process for the production of aromatic amines by catalytic hydrogenation of corresponding aromatic nitro compounds in the gas phase on immobilised catalysts which contain one or more metals of groups VIIIa, Ib, IIb, IVa, Va, VIa, IVb and Vb of the periodic system of elements (Mendeleyev) on a ceramic support material having a BET surface area of less than 40 m²/g, at molar ratios of hydrogen to nitro group or nitro groups of 3:1 to 30:1 and temperatures in the catalyst bed of 180 to 500°C, **characterised in that** loading of the catalyst with the aromatic nitro compounds used is increased continuously or step-wise from 0.01-0.5 to 0.7-5.0 kg/l × h, wherein maximum loading is achieved within 10 to 1000 hours.

2. Process for the production of aromatic amines according to claim 1, **characterised in that** the process is performed in reactors in which the heat transfer medium is located within the tubes and the catalyst is arranged outside the tubes containing the heat transfer medium.

3. Process for the production of aromatic amines according to claim 1, **characterised in that** the catalyst used is a supported catalyst, in which palladium had been applied onto graphite or coke containing graphite as the support, which has a BET surface area of 0.05 to 10 m²/g, and the palladium content of which catalyst is 0.1 to 7 wt.%, relative to the weight of the catalyst, in combination with a catalyst which contains one or more metals of groups VIIIa, Ib, IIb, IVa, Va, VIa, IVb and Vb on a ceramic support material having a BET surface area of less than 40 m²/g, and wherein the quantity ratio of ceramic supported catalyst to graphite supported catalyst is between 1/1 and 1/1000 parts by weight.

4. Process for the production of aromatic amines according to claims 1-3, **characterised in that** the catalyst beds are diluted with inert packing and optionally have activity gradients.

## Revendications

1. Procédé de préparation d'amines aromatiques par hydrogénation catalytique de composés nitro aromatiques correspondants en phase gazeuse sur des catalyseurs fixes qui contiennent un ou plusieurs métaux des groupes VIIIa, Ib, IIb, IVa, Va, VIa, IVb et Vb du système périodique des éléments (Mendeleïev) sur un matériau support céramique comportant une surface BET de moins de 40 m²/g, à des rapports molaires hydrogène à groupement nitro ou groupements nitro de 3:1 à 30:1 et à des températures de lit de catalyseur de 180 à 500°C,
**caractérisé en ce que** la charge du catalyseur sur des composés nitro aromatiques utilisés s'élève en continu ou par étapes à partir de 0,01 à 0,5 vers 0,7 à 5,0 kg/l x h, la charge maximale étant atteinte en 10 à 1 000 heures.

2. Procédé de préparation d'amines aromatiques selon la revendication 1, **caractérisé en ce que** le procédé s'effectue dans des réacteurs dans lesquels le caloporteur se trouve à l'intérieur des tuyaux et le catalyseur est agencé à l'extérieur des tuyaux contenant le caloporteur.

3. Procédé de préparation d'aminés aromatiques selon la revendication 1, **caractérisé en ce qu'**on utilise comme catalyseur un catalyseur supporté sur lequel on applique comme support du palladium sur graphite ou du coke contenant du graphite, qui possède une surface BET de 0,05 à 10 m²/g et dont la teneur en palladium est de 0,1 à 7% en poids, sur base du poids de catalyseur, en combinaison avec un catalyseur qui contient un ou plusieurs métaux des groupes VIIIa, Ib, IIb, IVa, Va, VIa, IVb et Vb sur un matériau support céramique avec une surface BET plus petite que 40 m²/g, et le rapport quantitatif de catalyseur supporté céramique à catalyseur supporté graphite étant entre 1/1 et 1/1 000 parties en poids.

4. Procédé de préparation d'amines aromatiques selon la revendication 1-3, **caractérisé en ce que** les lots de catalyseur sont dilués par des charges inertes ou présentent le cas échéant des gradients d'activité.
